# EUROPEAN PATENT SPECIFICATION

(11) **EP 2 793 022 B1**
(45) Date of publication and mention of the grant of the patent: **21.02.2018**
(21) Application number: 12856891.2
(22) Date of filing: 25.07.2012
(51) Int. Cl.: G01N 33/00, A23L 27/00

(54) **METHOD FOR EVALUATING FLAVOR IMPROVING AGENT**
VERFAHREN ZUR BEWERTUNG VON GESCHMACKSVERBESSERERN
PROCÉDÉ POUR ÉVALUER UN AMÉLIORATEUR DE GOÛT

(30) Priority: 16.12.2011 JP 2011276277
(43) Date of publication of application: 22.10.2014
(73) Proprietor: T. Hasegawa Co., Ltd., Chuo-ku Tokyo 103-8431 (JP)
(72) Inventor: SAITO Kana, Kawasaki-shi Kanagawa 211-0022 (JP); NAKAMURA Akio, Kawasaki-shi Kanagawa 211-0022 (JP); MATSUMOTO Tomona, Kawasaki-shi Kanagawa 211-0022 (JP); FUJIKI Ayano, Kawasaki-shi Kanagawa 211-0022 (JP)
(74) Representative: Cabinet Armengaud Aîné
(86) International application number: PCT/JP2012/068761
(87) International publication number: WO 2013/088773

(56) References cited:
- JP-A- H0 998 972
- JP-A- 2007 252 350
- JP-A- 2007 252 350
- JP-A- 2008 194 453
- JP-A- 2008 194 453
- JP-A- 2010 000 100
- JP-A- 2010 046 000
- JP-A- 2010 051 610
- JP-A- 2011 117 839
- US-A1- 2008 188 729
- SAITO-IIZUMI KANA ET AL: "Ethylmaltol Odor Enhances Salivary Hemodynamic Responses to Sucrose Taste as Detected by Near-Infrared Spectroscopy", CHEMOSENSORY PERCEPTION, SPRINGER NEW YORK LLC, US, vol. 6, no. 2, 27 April 2013 (2013-04-27), pages 92-100, XP035331794, ISSN: 1936-5802, DOI: 10.1007/S12078-013-9142-3 [retrieved on 2013-04-27]
- JANG IN-HWAN ET AL: "Biotransformation Process for the Production of Sotolon as a Natural Flavour Enhancer", HAN'GUG EUNG'YONG SAENGMYEONG HWA HAGHOEJI - JOURNAL OF THE KOREAN SOCIETY FOR APPLIED BIOLOGICAL CHEMISTRY, KOREAN SOCIETY FOR APPLIED BIOLOGICAL CHEMISTRY, SEOUL, KOREA, vol. 47, no. 1, 31 March 2004 (2004-03-31) , pages 49-54, XP053021061, ISSN: 1738-2203
- UHLIG J W ET AL: "Effect of phtalides on celery flavour", JOURNAL OF FOOD SCIENCE, WILEY-BLACKWELL PUBLISHING, INC, US, vol. 52, no. 3, 1 January 1987 (1987-01-01), pages 658-660, XP002536599, ISSN: 0022-1147, DOI: 10.1111/J.1365-2621.1987.TB06696.X
- BENEDETTI E ET AL: "SWEET AND BITTER TASTE: STRUCTURE AND CONFORMATIONS OF TWO ASPARTAME DIPEPTIDE ANALOGUES", JOURNAL OF PEPTIDE SCIENCE, JOHN WILEY AND SONS LTD, GB, vol. 1, no. 6, 1 January 1995 (1995-01-01) , pages 349-359, XP009027509, ISSN: 1075-2617, DOI: 10.1002/PSC.310010602
- KUROBAYASHI Y ET AL: "Flavour enhancement of chicken broth from boiled celery constituents", JOURNAL OF AGRICULTURAL AND FOOD CHEMISTRY, AMERICAN CHEMICAL SOCIETY, US, vol. 56, 29 December 2007 (2007-12-29), pages 512-516, XP002536596, ISSN: 0021-8561, DOI: 10.1021/JF072242P

## Description

### Technical Field

The present invention relates to a method for evaluating a flavor-improving agent. More specifically, the invention relates to a method for objectively evaluating the flavor-improving effect of a flavor-improving agent by previously selecting a subject that shows a change in signal strength to a known flavor-improving agent through measurement with an apparatus for in vivo optical measurement and then measuring a change in signal strength by a flavor-improving agent with the apparatus for in vivo optical measurement at a region around the temple area including the temple of the subject.

### Background Art

Conventionally, as methods for evaluating the flavor of foods and drinks, sensory evaluation depending on human senses has been mostly used. The sensory evaluation is also called a sensory inspection or a sensory test and evaluates an object with human senses (sight, hearing, taste, smell, touch). The sensory evaluation is suitable for evaluating comprehensive flavor, but has a disadvantage that subjective factors, such as individual differences, sensory exhaustion, and changes in physical conditions and mood, affect the evaluation and causes problems in reproducibility and objectivity. As a method in which objectivity is added to subjective sensory evaluation, quantitative description analysis (QDA) is known, but selection of common evaluative terms and training of panelists require large amounts of time and labor.

In addition, flavor evaluation with a device, such as various chromatographs including a liquid chromatograph, an odor sensor, or a taste sensor, is also employed. However, though the evaluation with a device such as a liquid chromatograph is objective, it requires analysis for each evaluation object item and takes a very long time to perform comprehensive evaluation. On the other hand, the evaluation with a sensor substituting for human smell or taste takes short time for the measurement, but is inferior in stability and reproducibility. In addition, the results do not necessarily agree with the results of sensory evaluation by subjects and are different from the actual senses of subjects in some cases. Thus, there is a problem in correlation with the results of sensory evaluation.

Accordingly, in order to objectify subjective sensory evaluation by human beings, it has been tried to employ a method of psychophysiology by observing and measuring a physiological response occurring in vivo instead of the conventional instrumental analysis and sensory evaluation. The psychophysiology is a new psychology field that studies emotional states and movements using a measurable biological reaction index, such as the size of the pupil, heart rate, blood pressure, electroencephalogram(EEG), magneto encephalography(MEG), cerebral blood flow, or stress hormone level, as a clue. A human changes the sense or emotion and, at the same time, shows physiological responses such as changes in blood pressure and heart rate and changes in stress substances in saliva by smelling something. Observation and measurement of these physiological responses are a method for evaluating flavor from an angle different from conventional instrumental analysis and sensory evaluation and are a new method for evaluating flavor.

In the apparatus for in vivo optical measurement known as an apparatus for measuring a physiological response, in vivo irradiation with light from visible light to near-infrared wavelengths is performed, the in vivo reflected or transmitted light is detected from near the surface of the living body, and an electrical signal corresponding to the light intensity is generated. The apparatus can noninvasively measure the real-time biological function (for example, see Patent Literatures 1 and 2).

In addition, recently, an apparatus for in vivo optical measurement (hereinafter referred to as "optical topography system") in which a subject is irradiated with light from a plurality of positions using optical fibers, in vivo transmitted light intensity is measured at a plurality of detection points, transmitted light intensity data is obtained for a relatively broad region including the detection points, and the response signals of cerebral activity are displayed as a time waveform (time course) or a two-dimensional image (topographic image), has been developed (for example, Patent Literature 3).

The in vivo optical measurements with these apparatuses are principally based on a relationship between a cerebral function and a relative change of a blood substance before and after application of a load such as stimulation (for example, cerebral activity of the visual cortex is detected by applying a visual stimulus) and is generally used for measuring a cerebral function. A specific cerebral site relates to the control of a specific function of a living body. The operation of the specific function changes the hemodynamics of the specific cerebral site. In the cerebral site at which neural activity is caused, expansion of capillary vessel, an increase of oxygenated hemoglobin, and a decrease of deoxygenated hemoglobin occur several seconds after the neural activity. The apparatus for in vivo optical measurement noninvasively measures the change in hemoglobin concentration. Hemoglobin in blood readily absorbs near-infrared light. This property allows detection of the blood flow in the cerebral cortex by irradiating the scalp with near-infrared light and detecting the reflected light and also thereby allows detection of the activity state.

The present inventors have focused on the characteristics of optical topography systems and have already disclosed a method for evaluating the optimum concentration of a taste substance with the optical topography system by preparing a plurality of aqueous solutions of the taste substance in different concentrations, giving the solutions to subjects, measuring a change in blood flow caused by the drinking of the solution, and determining the response strength of the change in blood flow and also have disclosed that an optimum concentration of a flavor-improving agent can be evaluated by adding the flavor-improving agent to an aqueous solution of the taste substance having a specific concentration lower than the optimum concentration to prepare a plurality of samples having different concentrations of the flavor-improving agent, giving the sample aqueous solutions to subjects, and measuring a change in blood flow caused by the drinking of the sample solution to determine the response strength of the change in blood flow (Patent Literature 4).

Conventionally, almost no apparatuses for in vivo optical measurement that measure a functional or physiological change in an organ other than the brain have been developed, but it has been reported on that the signal measured around the temple area with such an apparatus is based on salivary gland response associated with a taste stimulus, in particular, a change in blood flow accompanied by parotid gland activity, (Non-Patent Literature 1).

In clinical examination of a functional change of a salivary gland, which is an organ other than the brain, in general, MRI, ultrasonic echo, or scintigraphy is employed, but these technologies take long measurement times and are restrictive and invasive. Real-time monitoring of the functional change is also impossible. In general, in order to measure such salivary secretory activity, saliva spewed over several to several tens minutes is taken out with absorbent cotton placed in the oral cavity, which also does not allow real-time measurement of salivary gland secretory activity.

The change in saliva secretion capacity also relates to the oral cavity conditions (dry mouth, bad breath, difficulty in swallowing or chewing, and utterance) and the mental conditions. In order to avoid the influence by changes in these conditions, the measurement of saliva secretion capacity employs a method for spewing saliva spontaneously by biting absorbent cotton over several minutes as described above. Thus, it is difficult to measure the functional change of the salivary gland noninvasively at real time. Accordingly, it is required to develop a measuring method with a low restrictive apparatus. Against such a situation, in recent years, an apparatus for in vivo optical measurement of the salivary gland function at real time has been reported (Patent Literature 5), and it has been gradually revealed that the salivary gland function can be measured with an optical topography system, in particular the change in the brain activity which can be measured via the brain blood flow (Patent Literature 6).

### Citation List

### Patent Literature

[Patent Literature 1] Japanese Patent Laid-Open No. S57-115232
[Patent Literature 2] Japanese Patent Laid-Open No. S63-275323
[Patent Literature 3] Japanese Patent Laid-Open No. H09-98972
[Patent Literature 4] Japanese Patent Laid-Open No. 2010-46000
[Patent Literature 5] Japanese Patent Laid-Open No. 2010-100
[Patent Literature 6] Japanese Patent Laid-Open No. 2007-252350

### Non-Patent Literature

[Non-Patent Literature 1] J. Biomed. Opt., 16(4), 2011, 047002

### Summary of Invention

### Technical Problem

However, in conventional inventions, it is difficult to objectively evaluate the flavor-improving effect of some flavor-improving agents, for example, a flavor-improving agent that is an olfactory sense-stimulating substance, such as smell substance, and does not directly affect the salivary gland activity. Accordingly, there is a demand for a method, for example, that can evaluate a flavor-improving agent that shows a flavor-improving effect by being used together with a taste substance, even if the flavor-improving agent is an olfactory sense-stimulating substance, such as a smell substance that does not directly affect the salivary gland activity.

In view of such circumstances, the object of the present invention is to provide a method that can objectively evaluate the flavor-improving effect of not only a gustatory sense-stimulating substance but also a flavor-improving agent that is an olfactory sense-stimulating substance, such as a smell substance, of which evaluation for the flavor-improving effect by conventional technologies is difficult.

### Solution to Problem

The present inventors anticipated that in order to objectively evaluate the flavor-improving effect of a flavor-improving agent, it is effective to measure the change of a signal associated with the activity of the salivary gland that receives stimuli of the gustatory sense and olfactory sense by near-infrared spectroscopy and accordingly the change of the signal can be measured with the optical topography system described above, and the inventors have diligently studied. As a result, it was revealed that in the case of a flavor-improving agent that modifies the flavor by stimulating the gustatory sense, the change in blood flow around the temple area by drinking an aqueous solution of the flavor-improving agent after water varies depending on the concentration of the flavor-improving agent and thereby the flavor-improving effect of the flavor-improving agent can be properly evaluated by the amount of a change in blood flow, but in the case of a flavor-improving agent that modifies the flavor by stimulating the olfactory sense, the blood flow hardly varies in some subjects, whereas the blood flow varies depending on the concentration of the flavor-improving agent in some subjects. It was therefore revealed that if the amount of a change in blood flow around the temple area is merely measured with an optical topography system, the flavor-improving effect of, in particular, a flavor-improving agent acting on the olfactory sense is overlooked and proper evaluation of the flavor-improving effect of the flavor-improving agent is difficult.

Accordingly, the present inventors have further studied based on these findings and, as a result, have found that the flavor-improving effect of a flavor-improving agent, even if the flavor-improving agent is a type that acts on the olfactory sense, can be properly evaluated by giving to subjects a solution prepared by adding a known flavor-improving agent to an aqueous solution of a taste substance in a specific concentration and measuring a change in blood flow around the temple area caused by the drinking of the solution with an apparatus for in vivo optical measurement to previously selecting a subject that shows a change in signal strength by the change in blood flow, and then giving a test flavor-improving agent to the selected subject as an object and measuring the signal strength associated with the salivary gland activity by the drinking of the agent. Consequently, the present invention has been accomplished.

That is, the present invention relates to a method for evaluating a flavor-improving agent, the method comprising the steps of:
(1) giving subjects a solution prepared by adding a known flavor-improving agent to an aqueous solution of a taste substance selected from the group consisting of sweet substances, salty substances, acid substances, bitter substances, and umami substances and measuring a change in signal strength around the temple area caused by the drinking of the solution with an apparatus for in vivo optical measurement;
(2) selecting a subject showing a change in signal strength around the temple area by the addition of the known flavor-improving agent in the step (1); and
(3) giving a solution prepared by adding a test flavor-improving agent to an aqueous solution of the taste substance to the subject selected in the step (2) as an object, measuring a change in signal strength around the temple area caused by the drinking of the solution with an apparatus for in vivo optical measurement, and evaluating the flavor-improving effect from the change in signal strength as an index.

The present invention also relates to the method for evaluating a flavor-improving agent, wherein the measured signal is a change in amount of hemoglobin in blood occurred by irradiating the subject with near-infrared light with an apparatus for in vivo optical measurement.

The present invention also relates to the method for evaluating a flavor-improving agent, wherein the measured signal is a response signal of salivary gland activity.

The present invention also relates to the method for evaluating a flavor-improving agent, wherein the measured signal is a change in amount of hemoglobin associated with salivary gland activity.

The present invention also relates to the method for evaluating a flavor-improving agent, wherein the measured signal is a change in amount of hemoglobin associated with parotid gland activity.

The present invention also relates to a method for improving the flavor of a food or drink with a flavor-improving agent recognized to have a flavor-improving effect by the method for evaluating a flavor-improving agent.

The present invention also relates to an agent improving the flavor of a taste substance, the agent comprising an active ingredient selected by giving subjects a solution prepared by adding a known flavor-improving agent to an aqueous solution of a taste substance selected from the group consisting of sweet substances, salty substances, acid substances, bitter substances, and umami substances, measuring a change in signal strength around the temple area of each subject caused by the drinking of the solution with an apparatus for in vivo optical measurement, giving a solution prepared by adding any of candidate components to an aqueous solution of the taste substance to a subject showing a change in signal strength measured around the temple area as an object, and selecting a component showing a change in signal strength around the temple area caused by the drinking of the solution with an apparatus for in vivo optical measurement as the active ingredient. The flavor-improving agent, together with the taste substance, acts on a salivary gland and affects the activity of the salivary gland and thereby enhances or reduces the flavor of the taste substance, that is, the flavor-improving agent is a salivary gland-acting type.

### Advantageous Effects of Invention

The present invention can provide a method for evaluating a flavor-improving agent that can efficiently and objectively evaluate the flavor-improving effect of the flavor-improving agent noninvasively, low restrictively, in a short time.

### Brief Description of Drawings

Fig. 1 is a diagram illustrating measuring points in wearing a Hitachi ETG-4000 optical topography system. The channels in bold font (channels 33, 34, 43, 44, 40, 41, 51, 52) are those around the left and right temple area at which the response of a change in blood flow is particularly high,
Fig. 2 is an explanatory diagram illustrating the time schedule of conducting an experiment,
Fig. 3 is a sensory evaluation sheet used in the experiment,
Fig. 4 is a graph illustrating the amount of typical change in oxygenated hemoglobin with time after drinking of water and then a sample, around the temple area (channel 52),
Fig. 5 includes a graph (the upper) showing a relationship between the sample concentration and the response strength of a change in blood flow and a graph (the lower) showing a relationship between the strength of sample taste and the response strength of a change in blood flow, in 0 to 8% aqueous sugar solutions, around the temple area in all subjects,
Fig. 6 is a graph showing the measurement results of six subjects whose response strength of a change in blood flow was varied by addition of ethylmaltol to a 4% sugar solution, around the temple area of each subject,
Fig. 7 is a graph showing the measurement results of two subjects whose response strength of a change in blood flow was not varied by addition of ethylmaltol to a 4% sugar solution, around the temple area of each subject,
Fig. 8 is a graph showing blood flow measurement results when p-methoxycinnamaldehyde was added to a 4% sugar solution in one subject whose response strength of a change in blood flow was varied by addition of ethylmaltol in Example 2,
Fig. 9 is a graph showing blood flow measurement results when 2-phenylethyl alcohol was added to a 4% sugar solution in one subject whose response strength of a change in blood flow was varied by addition of ethylmaltol in Example 2,
Fig. 10 is a graph showing blood flow measurement results when citral was added to 0.1 % citric acid solution, and
Fig. 11 is a graph showing blood flow measurement results when soy sauce flavor was added to 0.5% saline.

### Description of Embodiments

In the method for evaluating a flavor-improving agent of the present invention, as described above, in the step (1), first, a solution prepared by adding a known flavor-improving agent to an aqueous solution of a taste substance selected from the group consisting of sweet substances, salty substances, acid substances, bitter substances, and umami substances is given to subjects; and then a change in signal strength around the temple area caused by the drinking of the solution is measured with an apparatus for in vivo optical measurement. The step (1) is a step for previously selecting subjects suitable for conducting evaluation by the method for evaluating a flavor-improving agent of the present invention and for investigating whether a change in signal strength can be detected with the apparatus for in vivo optical measurement by giving subjects a solution prepared by adding a known flavor-improving agent to an aqueous solution of a taste substance.

Drinking of a solution prepared by adding a flavor-improving agent to an aqueous solution of a taste substance stimulates a salivary gland. The stimulation of the salivary gland with the taste substance and the flavor-improving agent is transmitted to the brain through peripheral nerves to cause neural activity. In the brain in which the neural activity occurred, expansion of capillary vessel, an increase of oxygenated hemoglobin, and a decrease of deoxygenated hemoglobin occur several seconds after the neural activity. The apparatus for in vivo optical measurement measures the change in hemoglobin concentration and displays the change as a change in signal strength. That is, if the brain of a subject is irradiated with near-infrared light (700 to 1500 nm) from above the scalp with the apparatus for in vivo optical measurement, the near-infrared light passes through the brain tissue and is then reflected to return to the scalp. Since near-infrared light is absorbed by hemoglobin, the change in concentration of the intracerebral hemoglobin (change in blood flow with time) can be determined from a change in degree of attenuation of the reflected light with time. In the evaluation method of the present invention, the stimulation by the taste substance and the flavor-improving agent recognized by the brain is measured as a response signal of the salivary gland activity around the temple area, more specifically, as a change in amount of hemoglobin mainly associated with parotid gland activity.

The apparatus for in vivo optical measurement used in the evaluation method of the present invention irradiates near-infrared light, which has low photon energy and well passes through a human body, onto a scalp through an optical fiber. The light that has penetrated through the scalp and the skull and then has been reflected by the brain is converted into an electrical signal again on the scalp, and the electrical signal is then detected. A change in blood flow in the brain can be measured with the apparatus by measuring the intensity of the reflected light of near-infrared light absorbed by a chromoprotein contained in blood, hemoglobin. Among apparatuses for in vivo optical measurement, the evaluation method of the present invention preferably uses an optical topography system, i.e., an apparatus measuring the relative amount of change in hemoglobin that varies with the neural activity of the cerebral cortex at many points by near-infrared spectroscopy and displaying the results as a brain function image. Since it is known that the cerebral blood flow increases at the activated site, the use of an optical topography system allows completely simultaneous measurements of local changes in cerebral blood flow at many points and also allows observation of the cerebral activity as an image.

The optical topography system has a basic structure including an irradiation device of transmitting light irradiated from a near-infrared semiconductor laser to an optical fiber, an irradiation detector (probe) for arranging an irradiation fiber and a detection fiber at predetermined positions on a scalp, and a calculation unit for processing the detected optical signals and computing and displaying the processing result. The light reflected by the head enters a photodiode from the detection fiber and is converted into an electrical signal, and then the electrical signal is determined for the irradiation point from which the light irradiated. The change in concentration of hemoglobin is calculated based on the detected data of the optical intensity at each measurement point to generate a topography image.

A known flavor-improving agent can be evaluated by measuring a change in signal strength around the temple area of a subject with an apparatus for in vivo optical measurement and statistically processing the data of the blood flow at each channel (CH) corresponding to around the temple area including the temple. The optical topography system that can be used in the present invention is, for example, Hitachi ETG-4000 optical topography system (manufactured by Hitachi Medical Corporation: 52 channels).

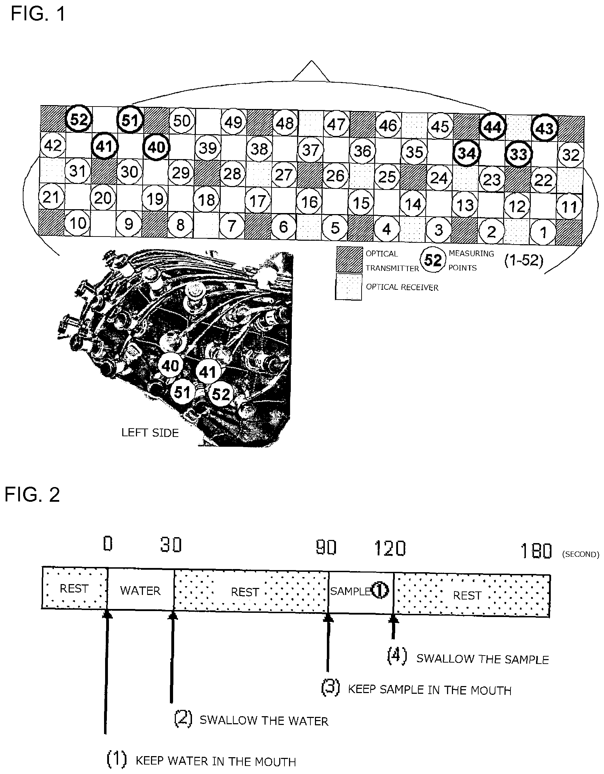

In the evaluation method of the present invention, the site for measuring the response strength of a change in blood flow is around the temple area including the temple, as described above. In this site, the signal associated with the activity of a salivary gland, in particular, of a parotid gland is measured at real time. The response of a change in blood flow at the temple of the forehead is measured with, when the Hitachi ETG-4000 optical topography system is used, for example, channels 20, 21, 30, 31, 40, 41, 42, 50, 51, and 52 on the left side and channels 11, 12, 22, 23, 32, 33, 34, 43, 44, and 45 on the right side. Among these channels, channels 33, 34, 43, 44, 40, 41, 51, and 52 (Fig. 1) are particularly preferred.

The taste substance used in the step (1), i.e., a sweet substance, a salty substance, an acid substance, a bitter substance, or an umami substance, is not particularly limited, and a conventionally known substance can be arbitrarily used. Examples of the sweet substance include saccharides such as sugar, glucose, fructose, and maltose; glycosides such as Glycyrrhiza extracts, stevia extracts, and Momordica grosvenori extracts; artificial sweeteners such as aspartame, saccharin, sucralose, and acesulfame potassium; and sweet-tasting proteins such as monellin and curculin.

Examples of the salty substance include sodium chloride and potassium chloride.

Examples of the acid substance include inorganic acids such as carbonic acid; and organic acids contained in, for example, lemons.

The bitter substances are of great variety, and examples thereof include substances used in foods, such as hop extracts (humulones), caffeine, Cinchona extracts (quinine), naringin, theobromine, Picrasma extracts, wormwood extracts, and Gentiana extracts; bitter substances contained in crude drugs, such as berberine of Coptis rhizome, swertiamarin of Swertia herb, quassin of Picrasma wood, gentiopicroside of Gentiana, and obakunone of Phellodendron; substances used in medicines, such as alkaloids; substances contained in foods, such as polyphenols (catechin, isoflavone, and chlorogenic acid); inorganic metal salts such as magnesium chloride; bitter terpenoids such as limonene contained in orange peel; and bitter peptides such as diketopiperazine isolated as a bitter substance generated in cheese. In addition, some of flavor components such as menthol and peppermint oil cause bitter aftertastes.

Examples of the umami substance include nucleic acids such as inosinic acid, guanylic acid, and adenylic acid; amino acids such as sodium glutamate, aspartic acid, alanine, glycine, arginine, serine, histidine, tricholomic acid, and ibotenic acid; parts of, for example, di-, tri-, and tetrapeptides; organic acids such as succinic acid, acetic acid, citric acid, and lactic acid; and combinations thereof.

The known flavor-improving agent may be any substance that shows a flavor-improving effect on the taste substance to which the agent is added.

Examples of known sweetness-improving agents include maltol, ethylmaltol, and sotolon.

Examples of known saltiness-improving agents include theanine, heated palatinose, dihydrochalcone acid, pyroglutamates, phthalides, and soy sauce flavor.

Examples of known acidity-improving agents include theanine, heated palatinose, and cyclodextrin.

Examples of known bitterness-improving agents include aspartame, sodium glutamate, and theanine.

Examples of known umami improving agents include nucleic acids such as inosinic acid and guanylic acid; sulfone group-containing compounds such as taurine; phosphates such as disodium phosphate; nontoxic salts of gluconic acid; and unsaturated N-alkamide.

The details of giving subjects a solution prepared by adding a known flavor-improving agent to an aqueous solution of a taste substance and measuring a change in signal strength caused by the drinking of the solution with an apparatus for in vivo optical measurement will be described in the paragraph of the step (3) below.

In the step (2), a subject showing a change in signal strength around the temple area by the addition of the known flavor-improving agent in the step (1) is selected.

The present inventors have found as described above that, in particular, when the flavor-improving agent used is a smell substance, a change in signal strength around the temple area can be easily detected with an apparatus for in vivo optical measurement in some subjects, whereas the change is hardly detected in some subjects. Accordingly, in the evaluation method of the present invention, a subject in which the effect by adding a known flavor-improving agent is detected is selected in the above-described steps (1) and (2), and in the subsequent step (3), a change in signal strength in the selected subject is measured by near-infrared spectroscopy to efficiently and objectively evaluate the flavor-improving effect of a flavor-improving agent that acts on the gustatory sense and olfactory sense. In the present invention, it is important to select a subject showing a change in the signal strength in the step (2). However, this does not mean that when a flavor-improving agent recognized to have a flavor-improving effect is actually applied to a subject who has not been selected in the step (2) by means of a food or drink, the flavor-improving effect is not recognized.

In the step (3), a solution prepared by adding a test flavor-improving agent to an aqueous solution of the taste substance is given to the subject selected in the step (2) as an object; a change in signal strength around the temple area caused by the drinking of the solution is measured with an apparatus for in vivo optical measurement; and the flavor-improving effect is evaluated by using the change in signal strength as an index. The aqueous solution of the taste substance used in the step (3) is the same as that used in the step (1).

Herein, the flavor-improving effect of a flavor-improving agent is an effect of increasing the strength of sensory flavor of a taste substance caused by the addition of the flavor-improving agent to a solution of a lower or higher concentration than the proper concentration of the taste substance.

In the evaluation method of the present invention, a larger change in signal strength can evaluate that the salivary gland activity is strong and the flavor-improving effect is high, and a smaller change in signal strength can evaluate that the salivary gland activity is weak and the flavor-improving effect is low. The test flavor-improving agent refers to a chemical substance or biological substance that is a target of the evaluation of a flavor-improving effect in the evaluation method of the present invention.

The step (3) can recognize a correlation between the results of sensory evaluation and the results of the evaluation method of the present invention by measuring a change in blood flow (change in signal strength) with an optical topography system worn by a subject during sensory evaluation of an aqueous solution of the taste substance by the subject. The evaluation of the flavor-improving agent can be performed by statistically processing the data relating to the blood flow at each channel (CH) of the optical topography system, as described above.

In the evaluation method of the present invention, an aqueous solution of a taste substance and a solution prepared by adding a (test) flavor-improving agent to an aqueous solution of the taste substance (hereinafter, both solutions are each referred to as a "sample") are given to a subject as an object (usually, samples containing different concentrations of the (test) flavor-improving agent are prepared, and the subject drinks all of them), and a change in signal strength around the temple area caused by the drinking of the solution is measured with an apparatus for in vivo optical measurement. This procedure is performed, for example, as follows.

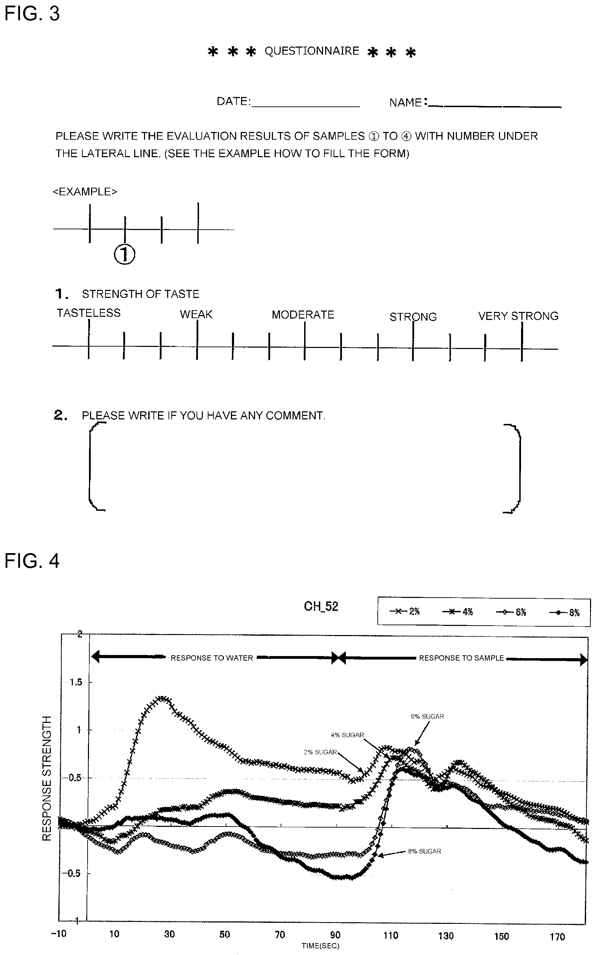

Each subject is held in a resting state, keeps water in the mouth such that the water spreads throughout the mouth for 30 seconds, then swallows the water, and then is held in a resting state for 60 seconds. Subsequently, the subject keeps a sample in the mouth to carefully taste the sample in the mouth for 30 seconds, then swallows the sample, and then is held in a resting state for 60 seconds again. Subsequently, the subject keeps water in the mouth again such that the water spreads throughout the mouth for 30 seconds, then swallows the water, and then is held in a resting state for 60 seconds. Subsequently, the subject keeps another sample in the mouth to carefully taste the sample in the mouth for 30 seconds, then swallows the sample, and then is held in a resting state for 60 seconds again. Thus, drinking of water and drinking of a sample are alternately repeated (Fig. 2). A sample can be evaluated at about four or five different concentrations in each experiment. In such a case, it is preferable to evaluate a sample at a lower concentration first, and the concentration of the sample is gradually increased. Samples at previously selected four or five different concentrations are sequentially evaluated by the above-described procedure. During the evaluation of the sample, including the times of drinking and resting, the blood flow is measured, and the change therein is recorded. In addition, sensory evaluation is performed during the tasting of the sample in the mouth for 30 seconds. After completion of the evaluation at a series of four or five concentrations, the strength of flavor of the sample is concluded lastly to fill the sensory evaluation sheet (Fig. 3).

When a sample is drunk in accordance with the above-described procedure, the blood flow around the temple area gradually increases after putting the sample into the mouth, and the signal strength reaches the maximum within about 5 to 30 seconds. Subsequently, the signal strength gradually decreases and reaches the original level within about 60 seconds after putting the sample into the mouth. Accordingly, a value obtained by subtracting the "minimum" blood flow within about 15 seconds after the moment of putting a sample into the mouth from the "maximum" blood flow during from about 5 to 30 seconds after putting the sample into the mouth at a specific site, i.e., the response strength of a change in blood flow, is defined as a change in signal strength around the temple area. The results of investigation by repeating the experiment demonstrate that the response strength pretty well agrees with the strength of the sensory flavor.

Examples of flavor-improving agents that can be evaluated by the method for evaluating a flavor-improving agent of the present invention include nonvolatile tasting agents and volatile smell substances. The flavor-improving agent may be any substance that has an effect of varying the flavor of the taste substance, for example, an effect of improving flavor by a flavor, an effect of improving the taste of a high-sweetness sweetener by a flavor such as sugar flavor, a synergistic effect of an umami substance by using an amino acid and a nucleic acid-based seasoning together, an effect of masking bitterness by a flavor, an effect of masking bitterness or astringency by a sweetener, an effect of overcoming the drawback of a high-sweetness sweetener by using another high-sweetness sweetener together, an effect of enhancing saltiness by an acid, or an effect of enhancing saltiness by a flavor. For example, addition of a flavor-improving agent having a flavor-enhancing effect to an aqueous solution of a taste substance in a lower concentration than the optimum concentration increases the sensory rating and exhibits a tendency to show a response strength equivalent to that of an aqueous solution of a taste substance in a concentration of the same as or higher than the optimum concentration.

An embodiment of the method for evaluating a flavor-improving agent of the present invention proposes a method for improving flavor by adding any flavor-improving agent to an aqueous solution of a taste substance in a lower or higher concentration than the optimum concentration. For example, in development of a drink, in order to decrease the sugar intake for reducing the caloric value, the sugar concentration is set to a level lower than the most preferable concentration from the point of flavor, and the flavor is compensated with a sugar flavor. In a soup, in order to decrease the salt intake, the salt concentration is set to a level lower than the most preferred concentration from the point of flavor, and the flavor is compensated with a saltiness enhancer.

### Examples

The present invention will now be more specifically described by examples.

Example 1: Investigation of taste strength of aqueous solution of taste substance showing basic taste and salivary gland response In this example, how the salivary gland response varies was investigated by giving aqueous solutions of a taste substance having a typical basic taste, sweetness, in different concentrations to a subject and measuring a change in blood flow around the temple area caused by the drinking of the solutions.

The samples, subjects, measuring apparatus, measuring method, and results are shown below.

### [Sample]

Sample 1: aqueous sugar solution: 0%, 2%, 4%, 6%, and 8%

### [Subject]

Arbitrarily selected eight subjects (males and females in their twenties to thirties)

### [Measuring apparatus]

Hitachi ETG-4000 optical topography system (manufactured by Hitachi Medical Corporation: 52 channels)

### [Measuring method]

A probe (Fig. 1) having a large number of sensors connected to an optical topography system body was set to the head of each subject, and then each sample was given to the subjects sequentially, followed by measurement. In accordance with the time schedule shown in Fig. 2, after resting, water was first given, and then a sample was given to aggressively evaluate the flavor. The order of drinking aqueous solutions of one kind of a taste substance given to the subjects was from a lower concentration to a higher concentration sequentially.

The sensory evaluation was performed in accordance with the evaluation questionnaire shown in Fig. 3 for the strength of taste as 0: tasteless, 3: weak, 6: moderate, 9: strong, and 12: very strong.

### [Results]

(1) Fig. 4 is a graph illustrating the amount of typical change in oxygenated hemoglobin with time when water was put in the mouth and drunk and then a sample was put in the mouth and drunk, measured with an optical topography system around the temple area (channel 52). In this graph, the response within 0 to 90 seconds is that to water, and the response within 90 to 180 seconds is that to the sample. The response strength was determined by that "when the time at which a sample was drunk is defined as a time of 0 second, (the maximum blood flow within 5 to 30 seconds) - (the minimum blood flow within 0 to 15 seconds)".
(2) Relationship between sample concentration and response strength

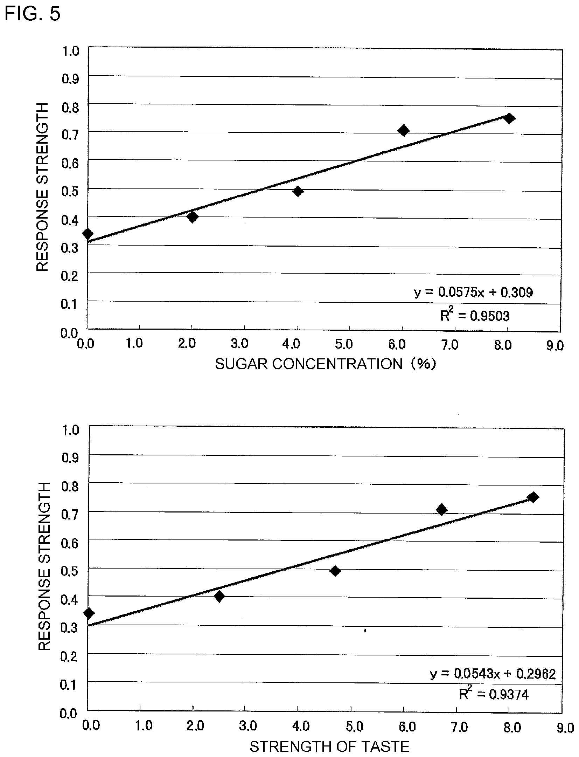

In the relationship between the sample concentration and the response strength measured around the temple area, the response strength to sugar solutions were larger than that to water in all subjects. In addition, in the investigated sugar concentration range (0 to 8%), an enhancement of the response strength depending on the sample concentration and the sweetness strength was observed. In particular, a positive correlation was observed between the sample concentration or the sweetness strength and the mean of the response strengths of changes in blood flow measured around the temple area of all subjects, to show high linearity (Fig. 5, R² value of (sample concentration) - (response strength): 0.95, R² value of (sweetness strength) - (response strength): 0.94).

The above results demonstrate that the response strength of a change in blood flow measured around the temple area when a subject drank aqueous solutions of a taste substance in different concentrations shows highly positive correlations with the sample concentration and the sweetness strength, and it is believed that the results show the response strength is effective for evaluation of salivary gland activity.

### Example 2: Investigation of sample solution prepared by adding known flavor-improving agent to aqueous solution of taste substance and salivary gland response

In this example, as the aqueous solution of the taste substance in a specific concentration lower than the optimum concentration, 4% aqueous sugar solution was selected from the candidate concentrations in Example 1. Furthermore, as a known flavor-improving agent, various concentrations of ethylmaltol (which is one of sugar flavors and is believed to show a sweetness-enhancing effect) were added to the aqueous solution. The resulting solutions were given to each subject, and the salivary gland response when each solution was drunk was measured with an optical topography system. The effect by the addition of the flavor-improving agent was evaluated from the resulting signal strength.

The samples, subjects, measuring apparatus, measuring method, and results are shown below.

### [Sample]

Sample 1: 4% aqueous sugar solution and ethylmaltol (0 ppm, 1 ppm, 5 ppm, and 10 ppm)

### [Subject]

Arbitrarily selected eight subjects (males and females in their twenties to thirties)

### [Measuring apparatus]

Hitachi ETG-4000 optical topography system (manufactured by Hitachi Medical Corporation: 52 channels)

### [Measuring method]

The samples of four different concentrations were subjected to sensory evaluation from a lower concentration of ethylmaltol to a higher concentration sequentially in accordance with the time schedule shown in Fig. 2 as in Example 1. The sensory evaluation sheet shown in Fig. 3 was filled.

### [Results]

### (1) Addition of ethylmaltol and sensory evaluation of strength of taste

The strength of sensual sweetness increased with the amount of ethylmaltol added to the 4% aqueous sugar solution.

### (2) Relationship between addition of ethylmaltol and signal strength

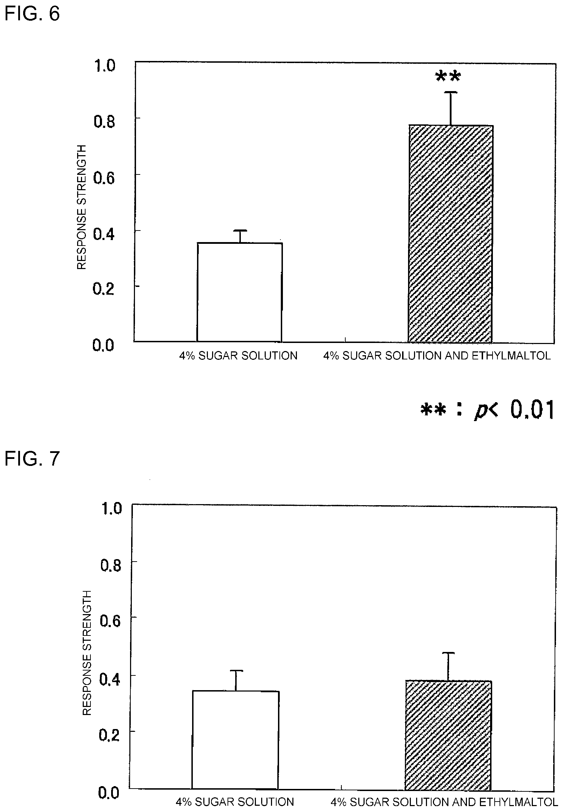

An enhancement in signal strength by addition of ethylmaltol was confirmed in six subjects at any concentration zone. That is, the subjects were classified into those showing a larger signal strength to a sample of the 4% aqueous sugar solution to which ethylmaltol was added than that to the 4% aqueous sugar solution alone and into those not showing such an enhancement (Figs. 6 and 7).

It is believed that the results above demonstrate that the effect of addition of a flavor-improving agent can be evaluated by a subject showing a change in signal strength in the measurement of salivary gland response when the subject drank a solution prepared by adding the flavor-improving agent to the aqueous solution of the taste substance in the specific concentration lower than the optimum concentration.

### Example 3: Investigation of sample solution prepared by adding flavor-improving agent of which flavor-improving effect is unknown to aqueous solution of taste substance and salivary gland response

In this example, various concentrations of a smell substance, as a flavor-improving agent, selected from p-methoxycinnamaldehyde, 2-phenylethyl alcohol, and citral was added to a 4% aqueous sugar solution. The resulting solutions were given to each subject showing a change in salivary gland response in Example 2, and the salivary gland response when each solution was drunk was measured with an optical topography system. The effect by the addition of the flavor-improving agent was evaluated from the resulting signal strength.

The samples, subjects, measuring apparatus, measuring method, and results are shown below.

### [Sample]

Sample 1: 4% aqueous sugar solution and p-methoxycinnamaldehyde (0 ppm, 1 ppm, 5 ppm, and 10 ppm) Samples 2 and 3: 4% aqueous sugar solution and 2-phenylethyl alcohol or citral (0 ppm, 1 ppm, 5 ppm, and 10 ppm)

### [Subject]

Subjects showed a change in salivary gland response in Example 2 (females in their twenties)

### [Measuring method]

### The same method as in Example 2

### [Results]

### (1) Addition of p-methoxycinnamaldehyde and sensory evaluation of strength of taste

The strength of sensory sweetness increased with the concentration of p-methoxycinnamaldehyde in the 4% aqueous sugar solution.

### (2) Relationship between addition of p-methoxycinnamaldehyde and signal strength

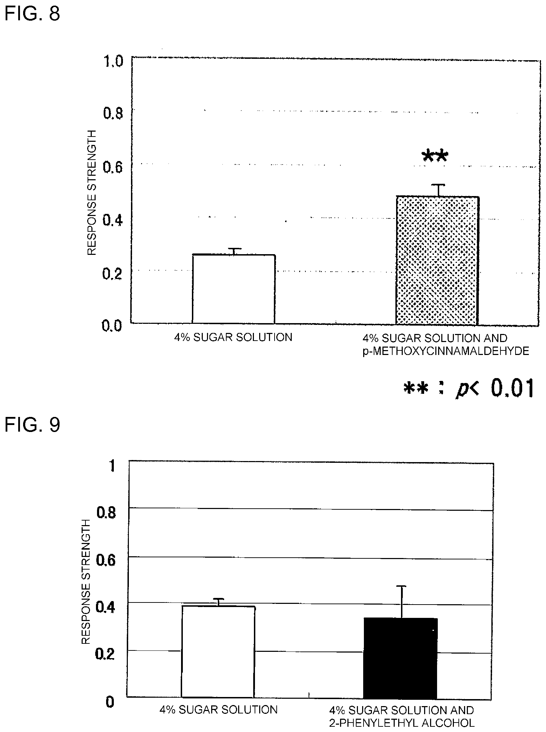

The results demonstrate that the response measured around the temple area was enhanced in the sample in which p-methoxycinnamaldehyde was added to a 4% aqueous sugar solution compared to that in the 4% aqueous sugar solution alone (Fig. 8).

### (3) Addition of 2-phenylethyl alcohol (or citral) and sensory evaluation of strength of taste

No increase of the strength of sensory sweetness was sensed even if 2-phenylethyl alcohol (or citral) was added to the 4% aqueous sugar solution.

### (4) Relationship between addition of 2-phenylethyl alcohol (or citral) and signal strength

The results demonstrate that the response measured around the temple area in the sample in which 2-phenylethyl alcohol (or citral) was added to a 4% aqueous sugar solution was similar to that in the 4% aqueous sugar solution alone (Fig. 9).

Example 4: Investigation of sample solution prepared by adding flavor-improving agent of which flavor-improving effect is unknown to aqueous solution of taste substance and salivary gland response In this example, a 0.1% aqueous citric acid solution was used as an aqueous solution of a taste substance in a specific concentration lower than the optimum concentration. Herein, various concentrations of citral, which did not show flavor-improving effect to sweetness, were added to a 0.1% aqueous citric acid solution as a flavor-improving agent. The resulting solutions were given to each subject showing a change in salivary gland response to a known flavor-improving agent, and the salivary gland response when each solution was drunk was measured with an optical topography system. The effect by the addition of the flavor-improving agent was evaluated from the resulting signal strength.

The samples, subjects, measuring apparatus, measuring method, and results are shown below.

### [Sample]

Sample 1: 0.1% aqueous citric acid solution and citral (0 ppm, 1 ppm, 5 ppm, and 10 ppm)

### [Subject]

Subjects showing a change in salivary gland response to a known flavor-improving agent (females in their twenties)

### [Measuring method]

### The same method as in Example 2

### [Results]

(1) Addition of citral and sensory evaluation of strength of taste The strength of sensory acidity increased with the concentration of citral in the 0.1 % aqueous citric acid solution.
(2) Relationship between addition of citral and signal strength

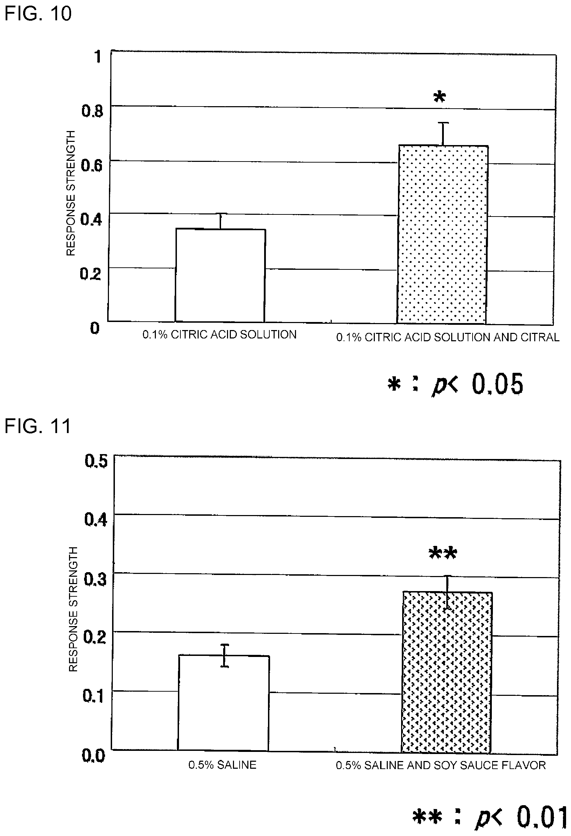

The results demonstrate that the response measured around the temple area was enhanced in the sample in which citral was added to a 0.1% aqueous citric acid solution compared to that in the 0.1% aqueous citric acid solution alone (Fig. 10).

### Example 5: Investigation of sample solution prepared by adding flavor-improving agent of which flavor-improving effect is known to aqueous solution of taste substance and salivary gland response

In this example, a 0.5% aqueous saline solution was used as an aqueous solution of a taste substance in a specific concentration lower than the optimum concentration. Herein, as a known flavor-improving agent, various concentrations of soy sauce flavor (which is believed to have a saltiness-enhancing effect, manufactured by T. Hasegawa Co., Ltd.) were added to a 0.5% aqueous saline solution. The resulting solutions were given to each subject, and the salivary gland response when each solution was drunk was measured with an optical topography system. The effect by the addition of the flavor-improving agent was evaluated from the resulting signal strength.

The samples, subjects, measuring apparatus, measuring method, and results are shown below.

### [Sample]

Sample 1: 0.5% aqueous saline solution and soy sauce flavor (0 ppm, 1 ppm, 5 ppm, and 10 ppm)

### [Subject]

Arbitrarily selected eight subjects (males and females in their twenties to thirties)

### [Measuring method]

### The same method as in Example 2

### [Results]

### (1) Addition of soy sauce flavor and sensory evaluation of strength of taste

The strength of sensory saltiness increased with the concentration of the soy sauce flavor in the 0.5% aqueous saline solution.

### (2) Relationship between addition of soy sauce flavor and signal strength

An enhancement in signal strength by addition of the soy sauce flavor was confirmed in some subjects at any concentration zone (Fig. 11). That is, the subjects were classified into two groups: a group consisting of six subjects showing enhanced signal strength to a sample of the 0.5% aqueous saline solution to which the soy sauce flavor was added compared to the 0.5% aqueous saline solution alone and a group consisting of two subjects not showing such an enhancement.

The above results revealed that the salivary gland response varies depending on the taste and smell constituting the flavor and that the salivary gland response increases with the concentration of taste. The change in blood flow was enhanced when a sugar solution, citric acid solution, or saline solution to which smell having a flavor-improving effect was added was drunk compared to that when sugar, citric acid, or sodium chloride was drunk alone, the focused salivary gland response was certainly varied by smell, and the modification by smell could be measured.

## Claims

1. A method for evaluating a test flavor-improving agent, **characterized in that** said method comprises the steps of:
(1) giving subjects a solution prepared by adding a known flavor-improving agent to an aqueous solution of a taste substance selected from the group consisting of sweet substances, salty substances, acid substances, bitter substances, and umami substances and measuring a change in signal strength around the temple area caused by the drinking of the solution with an apparatus for in vivo optical measurement;
(2) selecting a subject showing a change in signal strength around the temple area by the addition of the known flavor-improving agent in the step (1); and
(3) giving a solution prepared by adding a test flavor-improving agent to an aqueous solution of the taste substance to the subject selected in the step (2) as an object, measuring a change in signal strength around the temple area caused by the drinking of the solution with an apparatus for in vivo optical measurement, and evaluating the flavor-improving effect of the test flavor-improving agent from the change in signal strength as an index,
wherein the test flavor-improving agent is an olfactory sense-stimulating substance and
wherein the known flavor-improving agent is a substance that shows a flavor-improving effect on the taste substance to which the agent is added, and the test flavor-improving agent is a chemical substance or biological substance that is a target of the evaluation of a flavor-improving effect in the method.

2. The method for evaluating a test flavor-improving agent according to Claim 1, wherein the measured signal is a change in amount of hemoglobin in blood occurred by irradiating the subject with near-infrared light with an apparatus for in vivo optical measurement.

3. The method for evaluating a test flavor-improving agent according to Claim 2, wherein the measured signal is a response signal of salivary gland activity.

4. The method for evaluating a test flavor-improving agent according to Claim 2, wherein the measured signal is a change in amount of hemoglobin associated with salivary gland activity.

5. The method for evaluating a test flavor-improving agent according to Claim 2, wherein the measured signal is a change in amount of hemoglobin associated with parotid gland activity.

## Patentansprüche

1. Verfahren zum Bewerten eines Test-Geschmacksverbesserers, **dadurch gekennzeichnet, dass** das Verfahren die folgenden Schritte aufweist:
(1) Übergeben einer Lösung an Personen, wobei die Lösung durch Hinzufügen eines bekannten Geschmacksverbesserers zu einer wässrigen Lösung einer Geschmackssubstanz hergestellt ist, welche aus der Gruppe bestehend aus süßen Substanzen, salzigen Substanzen, sauren Substanzen, bitteren Substanzen und umami Substanzen ausgewählt ist, und Messen einer durch das Trinken der Lösung verursachten Veränderung der Signalstärke um den Schläfenbereich unter Verwendung einer Vorrichtung zur optischen In-vivo-Messung;
(2) Auswählen einer Person, die durch das in Schritt (1) erfolgte Hinzufügen des bekannten Geschmacksverbesserers eine Änderung der Signalstärke um den Schläfenbereich zeigt;
(3) Übergeben einer Lösung, die durch Hinzufügen eines Test-Geschmacksverbesserers zu einer wässrigen Lösung der Geschmackssubstanz hergestellt ist, an die in Schritt (2) als Objekt ausgewählte Person, die eine durch das Trinken der Lösung verursachte Veränderung der Signalstärke um den Schläfenbereich unter Verwendung einer Vorrichtung zur optischen In-vivo-Messung misst und den Geschmacksverbesserungseffekt des Test-Geschmacksverbesserers anhand der Änderung der Signalstärke als einem Index bewertet,
wobei der Test-Geschmacksverbesserer eine den olfaktorischen Sinn stimulierende Substanz ist, und
wobei der bekannte Geschmacksverbesserer eine Substanz ist, die einen Geschmacksverbesserungseffekt auf die Geschmackssubstanz hat, der das Mittel hinzugefügt wird, und wobei der Test-Geschmacksverbesserer eine chemische Substanz oder eine biologische Substanz ist, welche ein Ziel der Bewertung eines Geschmacksverbesserungseffekts in dem Verfahren ist.

2. Verfahren zum Bewerten eines Test-Geschmacksverbesserers nach Anspruch 1, bei welchem das gemessene Signal eine Veränderung der Menge an Hämoglobin im Blut ist, welche durch das Bestrahlen der Person mit Licht im Nahinfrarotbereich mittels einer Vorrichtung zur optischen In-vivo-Messung auftritt.

3. Verfahren zum Bewerten eines Test-Geschmacksverbesserers nach Anspruch 2, bei welchem das gemessene Signal ein Reaktionssignal der Speicheldrüsenaktivität ist.

4. Verfahren zum Bewerten eines Test-Geschmacksverbesserers nach Anspruch 2, bei welchem das gemessene Signal eine Veränderung der Menge an Hämoglobin in Verbindung mit der Speicheldrüsenaktivität ist.

5. Verfahren zum Bewerten eines Test-Geschmacksverbesserers nach Anspruch 2, bei welchem das gemessene Signal eine Veränderung der Menge an Hämoglobin in Verbindung mit der Ohrspeicheldrüsenaktivität ist.

## Revendications

1. Une méthode pour évaluer un agent test d'amélioration de la flaveur, **caractérisée en ce qu'**elle comprend les étapes de :
(1) donner à des sujets une solution préparée en additionnant un agent d'amélioration de la flaveur connu à une solution aqueuse de substance gustative choisie dans le groupe consistant en des substances sucrées, des substances salées, des substances acides, des substances amères et des substances umami et mesurer, avec un appareil pour des mesures optiques in vivo, un changement de la force du signal aux environs de la fosse temporale causé par la consommation de ladite solution ;
(2) sélectionner un sujet montrant un changement de la force du signal aux environs de la fosse temporale par addition de l'agent d'amélioration de la flaveur connu à l'étape (1); et
(3) donner une solution préparée en ajoutant un agent test d'amélioration de la flaveur à une solution aqueuse de substance gustative au sujet sélectionné à l'étape (2) comme objet, mesurer, avec un appareil pour des mesures optiques in vivo, un changement de la force du signal aux environs de la fosse temporale causé par la consommation de ladite solution et évaluer l'effet d'amélioration de flaveur de l'agent test d'amélioration de la flaveur en utilisant le changement de force du signal comme indicateur,
dans laquelle l'agent test d'amélioration de la flaveur est une substance stimulant le sens olfactif et
dans laquelle l'agent d'amélioration de la flaveur connu est une substance qui montre un effet d'amélioration de la flaveur sur la substance gustative à laquelle on a ajouté l'agent et l'agent test d'amélioration de la flaveur est une substance chimique ou biologique qui est une cible pour l'évaluation de l'effet d'amélioration de la flaveur dans la méthode.

2. Méthode pour évaluer un agent test d'amélioration de la flaveur selon la revendication 1, dans laquelle le signal mesuré est un changement de la quantité d'hémoglobine dans le sang obtenu en irradiant le sujet avec une lumière proche infrarouge à l'aide d' un appareil pour des mesures optiques in vivo.

3. Méthode pour évaluer un agent test d'amélioration de la flaveur selon la revendication 2, dans laquelle le signal mesuré est la réponse signal de l'activité de la glande salivaire.

4. Méthode pour évaluer un agent test d'amélioration de la flaveur selon la revendication 2, dans laquelle le signal mesuré est un changement de la quantité d'hémoglobine couplé avec l'activité de la glande salivaire.

5. Méthode pour évaluer un agent test d'amélioration de la flaveur selon la revendication 2, dans laquelle le signal mesuré est un changement de la quantité d'hémoglobine couplé avec l'activité de la glande parotide.
